(19) Europäisches Patentamt
European Patent Office
Office européen des brevets

(11) Publication number: **0 463 676 A1**

(12) **EUROPEAN PATENT APPLICATION**

(21) Application number: **91201487.5**

(51) Int. Cl.[5]: **C07C 69/675**, C07C 67/22

(22) Date of filing: **14.06.91**

(30) Priority: **22.06.90 IT 2074290**

(43) Date of publication of application:
**02.01.92 Bulletin 92/01**

(84) Designated Contracting States:
**AT BE CH DE DK ES FR GB GR LI LU NL SE**

(71) Applicant: **ENIMONT ANIC S.r.l.**
**Via Ruggero Settimo 55**
**I-90139 Palermo(IT)**

(72) Inventor: **Messina, Giuseppe**
**Via Perpignan 27**
**I-07041 Alghero-Sassari(IT)**
Inventor: **Chessa, Giovanna**
**Via Porcellana 13**
**I-07100 Sassari(IT)**
Inventor: **Lorenzoni, Loreno**
**Via Sassari 184**
**I-07046 Porto Torres-Sassari(IT)**
Inventor: **Mansani, Riccardo**
**Via Milano 20**
**I-07100 Sassari(IT)**

(74) Representative: **Roggero, Sergio et al**
**Ing. Barzanò & Zanardo Milano S.p.A. Via Borgonuovo 10**
**I-20121 Milano(IT)**

(54) **Process for the synthesis of alpha-hydroxy-esters.**

(57) A new process for the synthesis of the α-hydroxy-esters described by formula (1), by starting from the corresponding α-hydroxy-nitriles is disclosed, which comprises the following reaction steps:

1. formation of the 2-hydroxy-imino-methyl-ester hydrochloride by means of the reaction of the hydroxy-nitrile with anhydrous methanol in the presence of hydrochloric acid;
2. reaction of said 2-hydroxy-imino-ester hydrochloride with water.

EP 0 463 676 A1

The subject-matter of the instant invention is a novel process for the synthesis of α-hydroxy-esters having the following general formula:

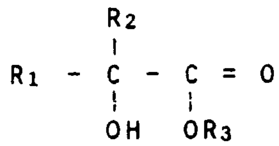

(1)

in which $R_1$, $R_2$ and $R_3$, which may be alike or different from one another, can be selected from among alkyl, aryl, arylalkyl radicals, which can be either branched or not.

The α-hydroxy-esters falling within the scope of the above general formula (1) are well-known compounds, widely described in the literature, which have found several applications at the industrial level. In particular, e.g., the compounds of formula (1), in which $R_1 = H$, $R_2 = CH_3$, $R_3$ = alkyl, i.e., the esters of lactic acid, display typical properties of cellulose or vinyl resin plasticizers, or, still, those compounds of formula (1) in which $R_1$, $R_2$ and $R_3$ are alkyl or arylalkyl radicals, represent useful intermediates in the synthesis of α-aminoacids.

The methods known from technical literature to prepare the α-hydroxy-esters of formula (1) are based on the use of pure α-hydroxy-acids.

The latter are either isolated from natural products, or prepared by reactions of hydrolysis of α-halo-acids, or of aldehyde or ketone cyanohydrin.

Anyway, both methods involve an additional step of separation of the hydroxy-acid from water. Such a separation is not always easy to be accomplished, because most α-hydroxy-acids, either undergo phenomena of decomposition during the distillation step, even under vacuum conditions, or, owing to the presence in the same molecule, of a hydroxy function and of a carboxy function, phenomena of self-esterification leading to the formation of linear polyesters [such as e.g., poly(lactic acid)]. An alternative method for the synthesis of the α-hydroxy-esters, in particular for the homologues of some important aminoacids, is disclosed in the paper by Velikov and co-workers (Preparation of ethyl esters of α-hydroxy-acids and gas-chromatography of the esters and α-hydroxy-nitriles - translated from Izvestiya Akademii Nauk SSSR, Scriya Khimicheskaya, No. 8, pages 1862-1864, August 1967), and is based on the reaction between α-hydroxy-nitriles, alcohols and hydrogen chloride gas. The execution procedure is quite laborious, and requires decidedly long reaction times (12-15 hours), during which the reaction mixture is submitted to the flow of a continuous HCl stream. Also the ester formation yields are never high, and reach high values only with increasing sizes of the alkyl radical.

The present Applicant found now, and such finding is the subject-matter of the present invention, that α-hydroxy-esters can be synthetized with high yields and within short reaction times, by causing the α-hydroxy-nitrile to react, in a first reaction step, with the acid in alcoholic solvents, and then submitting, in a second reaction step, the so obtained reaction product, deprived of the unreacted acid, to hydrolysis with a controlled water amount. Relatively to the method known from the prior art, high reaction yields were obtained in the reaction of formation of the α-hydroxy-ester, independently on the size of the alkyl radical of the hydroxy-nitrile used as the starting compound.

Furthermore, this process makes it possible an easier and cheap recovery to be accomplished of the α-hydroxyester from the reaction mixture.

In particular, it can be used in order to prepare compounds of formula:

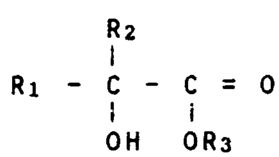

(1)

according to the following scheme:

Reaction 1:

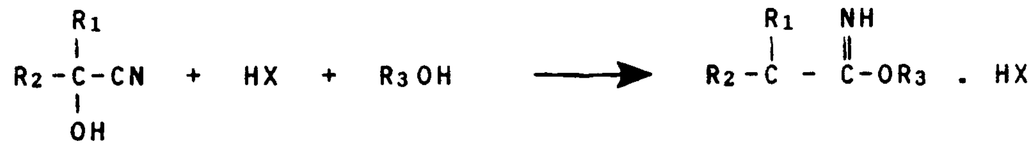

[Compound (I)]

Reaction 2:

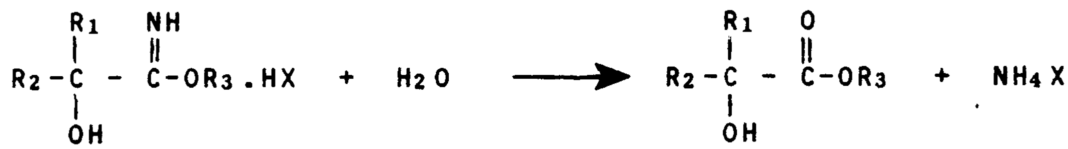

In practice, the reaction 1, formation of the α-hydroxy-imino-ether[compound (I)], is carried out by bringing the α-hydroxy-nitrile, dissolved in the suitable alcohol, into contact with the acid at temperatures comprised within the range of from 0 to 80 °C. The amount of acid used for each mol of hydroxy-nitrile is comprised within the range of from 1 to 10, preferably of from 1 to 2, whilst the amount of alcohol, per each mol of hydroxy-nitrile, is comprised within the range of from 2 to 10, preferably of from 4 to 5.

The compound (I) can be totally recovered from the reaction medium, by evaporating the excess of reactants, and then submitted to hydrolysis, reaction 2.

The reaction 2 can be carried out according to two different methods.

(1) The compound (I) is treated with an aqueous-alcoholic solution containing water in molar amounts equal to, or slightly larger, than of said compound (I). Water amounts comprised within the range of from 1 to 2 mol per each mol of compound (I) are to be preferred; larger amounts would involve, on the contrary, undesired phenomena of hydrolysis of the ester to yield carboxy acid, which reaction would be catalysed by the free acidity formed due to the acidic hydrolysis of ammonium chloride. According to this procedure, the reaction 2 can be carried out at a temperature comprised within the range of from 20 to 100 °C, preferably of from 40 to 60 °C, under atmospheric, or slightly superatmospheric, pressure.

(2) The reaction 2 can be carried out according to an alternative route, by treating the compound (I) with a large water excess; in this second case, the acidic hydrolysis of the ester is prevented by virtue of the strong dilution of the system. The hydrolysis temperatures are comprised within the range of from 20 to 100 °C, and preferably of from 25 to 60 °C.

The first method of hydrolysis is anyway to be preferred, in that it makes it possible an easier recovery to be accomplished of both the α-hydroxy-ester obtained, and of the ammonium chloride formed as a reaction by-product.

Particularly interesting α-hydroxy-nitriles for the purposes of the application of the instant invention are those falling within the scope of the formula

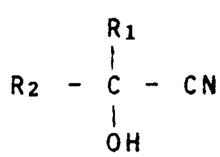

In our case, the use of acetone cyanhydrin proved to be particularly satisfactory. Particularly interesting alcohols for the purposes of the application of the present invention are the alcohols of formula

R-OH

in which R represents an alkyl or arylalkyl radical; among all, the reaction proceeds at best when methanol is used. At the end of the reaction, ammonium chloride is obtained, which separates as a solid phase, whilst the α-hydroxy-ester remains in solution.

Example No. 1

18.7 g (0.22 mol) of acetone cyanhydrin, 39.5 g (50 ml) of anhydrous methanol are charged to a jacketed, three-necked reactor of 100 ml of capacity, equipped with thermometer and gas inlet and outlet; the system is then adjusted at the temperature of 0° C and to it 23 g (0.631 mol) of hydrogen chloride gas is added at a flow rate of 23 mmol/minute. The so obtained mixture is kept with stirring at 0° C for a total time of 2 hours. After such period, the conversion of acetone cyanhydrin, monitored by GLC, is complete. Hydrogen chloride and the excess of methanol are then removed under vacuum, and 33.8 g (0.22 mol) of 2-hydroxy-2-methyl-imino-propionyl methyl ester hydrochloride (M.W. 153.45) is obtained as a white solid substance [Compound (I)].

I.R. Analysis (KBr): 1664 $cm^{-1}$; 1208 $cm^{-1}$.

The compound (I) is dissolved in 70 ml of methanol containing 4 g (0.22 mol) of water and 1.36 g of acetophenone (used as the internal standard for the computation of the reaction yield through GLC). The mixture is heated for 1.5 hours at methanol reflux temperature (60° C). After this heating period, the conversion of the iminoether [compound (I)] is complete. At the end of the reaction, the system is constituted by $NH_4Cl$, which separates, as a solid phase, at the reactor bottom, and a homogeneous, clear solution containing the methyl ester of 2-methyl-2-hydroxy-propionic acid. By filtration, 10.7 g (0.2 mol) of $NH_4Cl$ is obtained, whilst in the solution 24.26 g (0.205 mol) of the methyl ester of 2-hydroxy-2-methyl-propionic acid is present.

Example No. 2

By the procedure disclosed in Example 1, 33.8 g (0.22 mol) of compound (I) is prepared. To said compound (I), 60 ml of water and 0.509 g of acetophenone are then added. The system is kept with stirring at room temperature for 1 hour. After this period, 22.28 g (0.188 mol) of the methyl ester of 2-hydroxy-2-methyl-propionic acid is formed. After 2 and respectively 3 hours, the chromatographic analyses performed on the mixture, kept always stirred, do not show any changes in ester amount.

Example No. 3

13.1 g (0.185 mol) of 2-hydroxy-propionitrile and 27.66 g (35 ml) of anhydrous methanol are charged to a jacketed, three-necked reactor of 100 ml of capacity, equipped with thermometer and gas inlet and outlet; the system is then adjusted at the temperature of 0° C and to it 19.29 g (0.529 mol) of hydrogen chloride gas is added at a flow rate of 23 mmol/minute.

The mixture obtained in that way is kept with stirring at 0° C for a total time of 1 hour. i.e., the necessary time for the total conversion of 2-hydroxy-propionitrile to take place. Hydrogen chloride and the excess of methanol are then removed under vacuum, and 25.54 g (0.1845 mol) of 2-hydroxy-imino-propionyl methyl ester hydrochloride (M.W. 138.45) is obtained as a white solid substance.

The 2-hydroxy-imino-propionyl methyl ester hydrochloride is dissolved in 50 ml of methanol containing 4 g (0.22 mol) of water and 1.023 g of acetophenone (used as the internal standard). The mixture is heated for 30 minutes at methanol reflux temperature.

After this heating period, the conversion of 2-hydroxy-imino-propionyl methyl ester hydrochloride is complete.

The reaction mixture is then cooled, and by filtration, 8 g (0.15 mol) of $NH_4Cl$ is obtained; in the filtrate, 18.072 g (0.174 mol) of the methyl ester of 2-hydroxy-propionic acid is present.

**Claims**

1. Process for the synthesis of α-hydroxy-esters comprising causing the corresponding α-hydroxynitrile to react with the interesting alcohol in the presence of a hydrogen halide acid and submitting the so obtained product to hydrolysis, after the preliminary removal of any unreacted acid.

2. Process for the synthesis of α-hydroxy-esters according to the preceding claim, characterized in that the preliminary reaction between the hydroxy-nitrile and the alcohol is carried out at a temperature comprised within the range of from 0 to 80° C.

3. Process for the synthesis of α-hydroxy-esters according to claim 1, characterized in that, in the preliminary reaction between the hydroxy-nitrile, the alcohol and the haloide acid, the alcohol is

methanol and the haloide acid is hydrochloric acid.

European Patent Office

# EUROPEAN SEARCH REPORT

Application Number

EP 91201487.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CH - A - 125 959 (CANADIAN ELECTROPRODUCTS) * Totality * | 1,2 | C 07 C 69/675<br>C 07 C 67/22 |
| X | DE - C - 544 499 (IG-FARBENINDUSTRIE) * Totality * | 1-3 | |
| D,A | IZVESTIYA AKADEMII NAUK SSSR, no. 8, August 1967, SCRIYA KHIMINSKAYA * Pages 1862-1864 * | 1-3 | |
| | | | TECHNICAL FIELDS SEARCHED (Int. Cl.5) |
| | | | C 07 C 67/00<br>C 07 C 69/00 |

The present search report has been drawn up for all claims

| Place of search | Date of completion of the search | Examiner |
|---|---|---|
| VIENNA | 10-09-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)